# EUROPÄISCHE PATENTSCHRIFT

(11) **EP 0 689 408 B1**
(45) Veröffentlichungstag und Bekanntmachung des Hinweises auf die Patenterteilung: **18.12.1996**
(21) Anmeldenummer: 94911866.5
(22) Anmeldetag: 07.03.1994
(51) Int. Cl.: A61F 13/02

(54) **MEDIZINISCHES PFLASTERMATERIAL SOWIE VERFAHREN ZU SEINER HERSTELLUNG**
MEDICAL PLASTER MATERIAL AND PROCESS FOR ITS MANUFACTURE
MATERIAU POUR PANSEMENT MEDICAL ET PROCEDE POUR SA FABRICATION

(30) Priorität: 18.03.1993 DE 4308649
(43) Veröffentlichungstag der Anmeldung: 03.01.1996
(73) Patentinhaber: Lohmann GmbH & Co. KG, 56567 Neuwied (DE)
(72) Erfinder: TALKE, Volker, D-56581 Ehlscheid (DE); LUDWIG, Karin, D-56513 Neuwied (DE); REINHOLD, Karl-Heinz, D-53547 Hausen (DE); SEEGER, Kurt, D-56513 Neuwied (DE)
(74) Vertreter: Flaccus, Rolf-Dieter, Dr.
(86) Internationale Anmeldenummer: EP9400669
(87) Internationale Veröffentlichungsnummer: WO9421206

(56) Entgegenhaltungen:
- CH-A- 347 603
- DE-A- 2 736 952
- DE-A- 3 423 293
- DATABASE WPI Week 9021, Derwent Publications Ltd., London, GB; AN 90-161514 & JP,A,2 105 043 (FUJI PHOTO FILM) 17. April 1990

## Beschreibung

Die Erfindung betrifft ein medizinisches Pflastermaterial mit einem Träger, der in gleichmäßigem und porösem Auftrag mit einem Haftschmelzklebstoff beschichtet ist, sowie ein verfahren zu seiner Herstellung.

Medizinische Pflastermaterialien setzen sich nach dem Stand der Technik aus Trägermaterialien von textilen Flächengebilden und/oder Folien, beschichtet mit Klebemassen aus der Gruppe Naturkautschuk, Synthesekautschuk, Polyvinylether und Polyacrylatcopolymere auf Lösemittel- oder Dispersionsbasis zusammen.
Von diesen Klebemassen sind Naturkautschukkleber wegen ihrer hohen Allergisierungsrate sowie wegen ihrer geringen Alterungsbeständigkeit von Nachteil und wurden infolgedessen bei der medizinischen Anwendung immer mehr in den Hintergrund gedrängt. Dispersionsklebemassen weisen den Nachteil auf, daß wegen ihrer geringen Feuchteresistenz eine Transpiration der Haut zum Anlösen der Klebemassen führt, weshalb auch diese sich am Markt nicht durchsetzen konnten. Weiterhin haben die derzeit meist gebräuchlichen Lösemittelacrylatkleber den Nachteil, daß das verwendete Lösemittel unter Einsatz von hohem Energie- und Maschinenaufwand verdampft und danach entweder verbrannt oder auch zurückgewonnen und entsorgt werden muß. Die CH-A-347,603 offenbart einen Herstellungsprozeß für medizinisches Pflastermaterial wobei das Trägermaterial eine Wasserdampfdurchlässigkeit von mindestens 2000 g/m²·24h bei 40°C und 90% rel. LF aufweist. Dieses Dokument bildet den Oberbegriff des Anspruchs 1. Aus der DE-A-2736952 sind Schmelzhaftkleber auf Basis von Blockcopolymerisaten bekannt.
Moderne Haftschmelzklebestoffe aus Synthesekautschukklebern auf der Basis von Blockcopolymeren erlangten bisher keine Marktrelevanz, weil ihre geringe Wasserdampfdurchlässigkeit eine zuverlässige Haftung über einen längeren Zeitraum auf der menschlichen Haut, die im Ruhezustand ca. 500 g/m²/24 h Wasser abgibt, verhindert.

Eine Erhöhung der Wasserdampfdurchlässigkeit bei einem medizinischen Pflastermaterial mittels porösem Kleberauftrag beispielsweise unter Verwendung von Siebdruckauftragswerken ist für medizinische Anwendungen von Pflastermaterialien bekannt. Jedoch weisen auch derart beschichtete medizinische Pflastermaterialien, bei welchen der Kleber nur inselförmig und nicht zusammenhängend aufgetragen wird, den Nachteil auf, daß bei solcherart konfektionierten Pflastern aus Folien keine Bakteriendichtigkeit garantiert werden kann, und daß es darüberhinaus bei textilen Flächengebilden zum Ausfransen einzelner Fäden kommt, was nicht nur optisch stört, sondern bei elastischen Trägermaterialien fallweise eine zuverlässige Anwendung unmöglich macht.
Darüberhinaus haben mittels Siebdruck aufgetragene Haftschmelzklebstoffe die Eigenschaft, daß ein halbkugelförmiger und kein zylindrischer Auftrag erzielt wird. Dies hat zur Folge, daß bei Direktbeschichtungen die zur Haut hin klebende Fläche im Vergleich zur Kleberauftragsmenge sehr klein und abhängig vom Applikationsdruck ist, und daß bei Transferbeschichtung auch bei hohem Kaschierdruck oftmals keine ausreichende Haftung auf den Trägermaterialien erreicht wird.
Andererseits erreichten Haftschmelzkleberaufträge mit zusammenhängender Klebemasse und inselförmig angeordneten klebefreien Stellen, sofern diese mittels Tiefdrucktechnik erzeugt wurden, deshalb bei der medizinischen Anwendung keine Bedeutung, weil beim Tiefdruck nur Kleberaufträge bis 30 g/m² möglich waren. Bei höherem Auftrag wurden keine zufriedenstellenden Feinstrukturen erreicht und die erforderliche Porosität wurde drastisch verringert. Diese vergleichsweise geringen Kleberaufträge führten zudem zu einer niederen inneren Elastizität, die nicht mehr ausreichend war, um durch Bewegungen hervorgerufene Flächendifferenzen zwischen der menschlichen Haut und dem Trägermaterial auszugleichen. Somit wurde bei geringen Kleberauftragsmengen regelmäßig ein Ablösen der Randzonen des Pflastermaterials beobachtet.

Der vorliegenden Erfindung liegt die Aufgabe zugrunde, ein medizinisches Pflastermaterial der im Oberbegriff von Anspruch 1 genannten Art sowie ein Verfahren zu dessen Herstellung anzugeben, welche es unter Vermeidung der vorgenannten Nachteile und technischen Grenzen ermöglichen, Haftschmelzklebstoffe auf der Basis von Blockcopolymeren mit geeigneter Viskosität mittels Tiefdrucktechnik so auf Trägermaterialien von textilen Flächengebilden und/oder Folien zu beschichten, daR bei ausreichender Porosität die Menge des aufgetragenen Klebstoffes genügt, um auch bei Bewegungen eine zuverlässige und dauerhafte Haftung der Pflastermaterialien auf der menschlichen Haut zu gewährleisten.

Zur Lösung der Aufgabe schlägt die Erfindung bei einem medizinischen Pflastermaterial mit einem Träger, der in gleichmäßigem und porösem Auftrag mit einem Haftschmelzklebstoff beschichtet ist, vor, daß der mittels eines Tiefdruckauftragswerkes aufgetragene Haftschmelzklebstoff so auf dem Träger angeordnet ist,
a) daß er in Längs- und Querrichtung zusammenhängende Stege bildet, welche inselförmige klebstofffreie Flächenteile einschließen;
b) daß der Anteil der klebstofffreien Flächenteilen zwischen 30 und 60%, bevorzugt zwischen 40 und 57% der Gesamtfläche liegt und,
c) daß das Auftragsgewicht des Klebstoffs zwischen 30 und 160 g/m², bevorzugt zwischen 40 und 120 g/m² liegt.

Mit Vorteil gelingt durch die erfindungsgemäße Art des Auftrages eines Haftschmelzklebstoffes auf der Basis von Blockcopolymeren mit geeigneter Viskosität auf die vorgenannten Trägermaterialien, daß die beim Siebdruck beschriebenen Nachteile nicht auftreten, daß weiterhin eine durchaus befriedigende Porosität der klebstofffreien Flächenteile zwischen 30 und 60% erreicht und zudem eine zuverlässige Haftung des Pflastermaterials auf der menschlichen Haut auch bei durch Bewegungen hervorgerufenen Flächendifferenzen sich ergebende Spannungen gewährleistet ist.

Eine vorteilhafte Ausgestaltung sieht vor, daß die im Haftschmelzklebstoff regelmäßig angeordneten, klebstofffreien Inseln einen Abstand von höchstens 3 mm voneinander haben. Hierfür sieht eine Ausgestaltung vor, daß die aufgetragenen Kleberstege an ihrer Basis auf dem Trägermaterial maximal 3 mm breit sind.

Der Haftschmelzklebstoff ist ein Blockcopolymer mit einer Viskosität bei 120 bis 180 °C zwischen 1.000 und 20.000 mPa·s, bevorzugt zwischen 2.000 und 10.000 mPa·s.

Eine Ausgestaltung sieht vor, daß das Trägermaterial ein textiles Flächengebilde ist. Ein solches Flächengebilde weist eine vergleichsweise hohe und durch die textile Struktur einstellbare Porosität auf. Das Trägermaterial kann aber auch eine dampfdurchlässige Folie sein.

Das Pflastermaterial weist eine Wasserdampfdurchlässigkeit von mehr als 2.000 g/m²/24 h auf.
Bei dem medizinischen Pflastermaterial kann es sich um ein Heftpflaster, eine Pflasterbinde, einen Wundschnellverband, ein Fixierpflaster, ein Allergietestpflaster oder eine bakteriendichte Wundauflage handeln.
Bei dem klebstoffbeschichteten Träger kann es sich auch um ein außerhalb des medizinischen Bereichs verwendbares Klebmaterial wie Klebeband, Etiketten etc. handeln. Bei diesem ergibt sich gegenüber vollflächig beschichteten Klebematerialien eine bis zu 50%-ige Einsparung an Klebstoff.
Weiterhin ist vorgesehen, daß die Pflaster nach der Erfindung sterilisierbar sind. Dies kann in einer dem Fachmann bekannten Art und Weise durch Strahlung oder Ethylenoxid erreicht werden.

Ein Verfahren zur Herstellung des Pflastermaterials nach der Erfindung sieht vor, daß ein textiles Flächengebilde und/oder eine dampfdurchlässige Folie mittels eines beheizbaren Tiefdruckauftragswerkes, das bei der Klebstoffaufnahme sowohl mit einer starren als auch mit einer elastischen Rakel abgerakelt ist, mit Haftschmelzklebstoff auf Basis von Blockcopolymeren so porös beschichtet wird, daß die Klebemasse in Längs- und Querrichtung zusammenhängende Stege bildet, die klebstofffreie inselförmige Flächenteile einschließen, welche einen Abstand bis zu 3 mm voneinander aufweisen, wobei der Anteil der klebstofffreien Flächenteile zwischen 30 und 60% und bevorzugt zwischen 40 und 57% der Gesamtfläche liegt und die Klebestege an ihrer Basis maximal 3 mm breit sind, und daß das Pflastermaterial eine Wasserdampfdurchlässigkeit von mindestens 2.000 g/m²/24 h aufweist. Die Wasserdampfdurchlässigkeit wird bei 40°C und einer Differenz der relativen Luftfeuchte von 80% bestimmt.

Die Klebmasse wird mit einem Auftragsgewicht zwischen 30 und 160 g/m², bevorzugt zwischen 40 und 120 g/m² auf den Träger aufgebracht. Und schließlich ist vorgesehen, daß als Klebstoff ein Blockcopolymer mit einer Viskosität bei 120 bis 180 °C zwischen 1.000 und 20.000 mPa·s, bevorzugt zwischen 2.000 und 10.000 mPa·s verwendet wird.
Im folgenden wird die Erfindung anhand zweier Beispiele weiter verdeutlicht:

### Beispiel 1:

Eine 50 µm dicke silikonisierte Polyesterfolie der Fa. Daubert wird mittels eines beheizbaren Tiefdruckauftragswerkes, dessen Gravurwalze ein regelmäßiges, rautenähnliches Muster mit einer Gravurtiefe von 100 µm aufweist und bei der die in einem Abstand von 1,3 mm angeordneten, klebstofffreien Flächen 50% der Gesamtfläche belegen, mit 60 g/m² des Haftschmelzklebstoff Lunamelt PS 3785, der bei 150°C eine Viskosität von 13.740 mPa·s besitzt, bei einer Auftragstemperatur von 140°C beschichtet. Unmittelbar nach der Beschichtung wird unter 2 bar Druck eine 25 µm dicke Polymerfolie der Fa. ACE zukaschiert, die unbeschichtet eine Wasserdampfdurchlässigkeit von 4.400 g/m²/24 h besitzt. Die so beschichtete Folie weist eine Wasserdampfdurchlässigkeit von 2.500 g/m²/24 h auf und ist bakteriendicht. Die Klebkraft der zu Wundauflagen konfektionierten und γ-sterilisierten Muster beträgt nach AFERA 4001 15 N/25mm. Die Wundauflagen zeigen in Tragetests auf dem Rücken von 10 Probanden eine zuverlässige Haftung über 24 h.

### Beispiel 2:

Die im Beispiel 1 genannte Polyesterfolie wird mit einer Gravurwalze, bei der die klebstoffreie Fläche, die 44% der Gesamtfläche ausmacht, aus regelmäßigen Sechsecken besteht, und die eine Gravurtiefe von 140 µm aufweist, mit 95 g/m² des Haftschmelzklebstoffes H 2322 H01 der Fa. Findley Adhesives Inc., der bei 177°C eine Viskosität von 7.800 mPa·s aufweist, beschichtet. Unmittelbar nach der Beschichtung wird unter 4 bar Druck ein guerelastischer Pflasterstoff der Fa. TEN CATE MEDICAL B.V. zukaschiert. Nach Konfektionierung zu Wundschnellverbänden ist auch bei Dehnung kein Ausfransen der Pflasterränder zu beobachten. Die Pflaster hafteten bei Tragetests am linken Unterarm von 10 Probanden zuverlässig über 24 h. Die Wasserdampfdurchlässigkeit der Pflaster betrug 4.500 g/m²/24 h.

## Patentansprüche

1. Medizinisches Pflastermaterial mit einem Träger, der in gleichmäßigem und porösem Auftrag mit einem Haftkleber in der Weise beschichtet ist, daß er
a) in Längs- und Querrichtung zusammenhängende Stege bildet, welche inselförmige, klebstofffreie Flächenteile einschließen;
b) daß der Anteil der klebstofffreien Flächenteile zwischen 30 und 60%, bevorzugt zwischen 40 und 57% der Gesamtfläche liegt, dadurch gekennzeichnet,
- daß der Haftkleber ein Haftschmelzklebstoff auf Basis von Blockcopolymeren mit einer Viskosität bei 120 bis 180 °C zwischen 1.000 und 20.000 mPa·S, bevorzugt zwischen 2.000 und 10.000 mPa·S ist,
- daß der Haftschmelzkleber auf den Träger mittels Tiefdrucktechnik aufgebracht ist,
- daß das Pflastermaterial eine Wasserdampfdurchlässigkeit von mehr als 2.000 g/m²/24h aufweist, und
- daß das Auftragsgewicht des Klebstoffs zwischen 30 und 160 g/m², bevorzugt zwischen 40 und 120 g/m² liegt.

2. Medizinisches Pflastermaterial nach Anspruch 1, dadurch gekennzeichnet, daß die im Haftschmelzklebstoff regelmäßig angeordneten, klebstofffreien Inseln einen Abstand von höchstens 3 mm voneinander haben.

3. Medizinischen Pflastermaterial nach Anspruch 1, dadurch gekennzeichnet, daß die Kleberstege an ihrer Basis maximal 3 mm breit sind.

4. Medizinisches Pflastermaterial nach Ansprüchen 1 bis 4, dadurch gekennzeichnet, daß das Trägermaterial ein textiles Flächengebilde ist.

5. Medizinisches Pflastermaterial nach Anspruch 1, dadurch gekennzeichnet, daß das Trägermaterial eine dampfdurchlässige Folie ist.

6. Medizinisches Pflastermaterial nach Ansprüchen 1 bis 7, dadurch gekennzeichnet, daß es sich bei dem medizinischen Pflastermaterial um ein Heftpflaster, eine Pflasterbinde, einen Wundschnellverband, ein Fixierpflaster, ein Allergietestpflaster oder eine bakteriendichte Wundauflage handelt.

7. Medizinisches Pflastermaterial nach Anspruch 8, dadurch gekennzeichnet, daß die Pflaster sterilisierbar sind.

8. Verfahren zur Herstellung eines medizinischen Pflastermaterials nach Anspruch 1 bis 9, dadurch gekennzeichnet, daß ein textiles Flächengebilde und/oder eine dampfdurchlässige Folie mittels eines beheizbaren Tiefdruckauftragswerkes, das bei der Klebstoffaufnahme sowohl eine starre als auch eine elastische Rakel verwendet, mit Haftschmelzklebstoff auf Basis von Blockcopolymeren porös so beschichtet wird, daß die Klebmasse in Längs- und Querrichtung zusammenhängende Stege bildet, die klebstofffreie inselförmige Flächenteile einschließen, die einen Abstand bis zu 3 mm voneinander aufweisen, wobei der Anteil der klebstofffreien Flächenteile zwischen 30 und 60 %, bevorzugt zwischen 40 und 57 % der Gesamtfläche liegt und die Kleberstege an ihrer Basis maximal 3 mm breit sind, und daß das Pflastermaterial eine Wasserdampfdurchlässigkeit von mindestens 2.000 g/m²/24 h unter Meßbedingungen aufweist, wobei das Auftragsgewicht des Klebstoffs zwischen 30 und 160 g/m², bevorzugt zwischen 40 und 120 g/m² liegt.

9. Verfahren nach Anspruch 10 oder 11, dadurch gekennzeichnet, daß als Klebstoff ein Blockcopolymer mit einer Viskosität bei 120-180 °C zwischen 1.000 und 20.000 mPa·s, bevorzugt zwischen 2.000 und 10.000 mPa·s verwendet wird.

## Claims

1. Medical patch material having a support coated with an even and porous spread of a pressure-sensitive adhesive in such a manner that it
a) forms coherent webs in the lengthwise and transverse direction, which include island-shaped adhesive-free areas;
b) that the proportion of the adhesive-free areas amounts to between 30 and 60%, preferably between 40 and 57% of the total surface, characterized in that
- the pressure-sensitive adhesive is a pressure sensitive hot-melt adhesive based on block copolymers having a viscosity at 120 to 180°C of between 1,000 and 20,000 mPa·s, preferably between 2,000 and 10,000 mPa·s.,
- the pressure sensitive hot-melt adhesive is applied on the supporting material by means of gravure printing,
- the patch material has a water-vapor permeability of more than 2,000 g/m²/24 h, and
- that the coat weight of the adhesive amounts to between 30 and 160 g/m², preferably between 40 and 120 g/m².

2. The medical patch material according to claim 1 characterized in that the adhesive-free islands regularly arranged in the pressure sensitive hot-melt adhesive have a maximum distance of 3 mm to one another.

3. The medical patch material according to claim 1 characterized in that the base of the adhesive webs has a maximum width of 3 mm.

4. The medical patch material according to claims 1 to 3 characterized in that the supporting material is a textile fabric.

5. The medical patch material according to claim 1 characterized in that the supporting material is a vapor-permeable film.

6. The medical patch material according to claims 1 to 5 characterized in that the medical patch material is an adhesive patch, a patch-type bandage, an adhesive dressing, a securing patch, an allergy-test patch, or a bacteria-proof wound dressing.

7. The medical patch material according to claim 6 characterized in that the patches are sterilizable.

8. A process for the production of a medical patch material according to claims 1 to 7, characterized in that a textile fabric and/or a vapor-permeable film is coated with a pressure sensitive hot-melt adhesive based on block copolymers by means of a heatable direct gravure print coater, which uses both a rigid and an elastic coating knife in adhesive uptake, in such a porous manner that the adhesive substance forms coherent webs in the longitudinal and transverse direction, which include adhesive-free, island-shaped areas having a distance of up to 3 mm to one another, the proportion of the adhesive-free areas amounting to between 30 and 60%, preferably between 40 and 57% of the total surface and the base of the adhesive webs having a maximum width of 3 mm, and that the patch material has a water-vapor permeability of at least 2,000 g/m²/24 h under measuring conditions, the coat weight of the adhesive amounting to between 30 and 160 g/m², preferably between 40 and 120 g/m².

9. The process according to claim 8, characterized in that a block copolymer having a viscosity at 120-180°C of between 1,000 and 20,000 mPa·s, preferably between 2,000 and 10,000 mPa·s is used as adhesive.

## Revendications

1. Emplâtre ou timbre médical avec un support qui est recouvert d'une couche uniforme et poreuse avec une colle de contact, en une manière telle que
a) il forme en direction longitudinale et transversale des nervures cohérentes qui enferment des parties planes ou plates sans colle, en forme d'îlots,
b) la fraction des parties plates ou planes sans colle forme de 30 à 60%, de préférence, de 40 à 57% de la surface totale,
caractérisé en ce que
- la colle de contact est une colle de contact fusible à base de copolymères séquencés présentant une viscosité, à 120-180°C, comprise entre 1.000 et 20.000 mPa.s, de préférence, comprise entre 2.000 et 10.000 mPa.s,
- la colle de contact fusible est appliquée sur le support, à l'aide d'une technique d'héliogravure ou rotogravure,
- l'emplâtre ou timbre présente une perméabilité à la vapeur d'eau supérieure à 2.000 g/m²/24 h et
- le grammage de colle appliquée se situe entre 30 et 160 g/m², de préférence, entre 140 et 120 g/m².

2. Emplâtre ou timbre médical suivant la revendication 1, caractérisé en ce que dans la colle de contact fusible, des îlots régulièrement agencés, sans colle, ont une distance mutuelle d'au plus 3 mm.

3. Emplâtre ou timbre médical suivant la revendication 1, caractérisé en ce que les nervures de colle ont une largeur maximale de 3 mm, à leur base.

4. Emplâtre ou timbre médical suivant les revendications 1 à 3, caractérisé en ce que le matériau de support est un article plat textile.

5. Emplâtre ou timbre médical suivant la revendication 1, caractérisé en ce que le matériau de support est une feuille perméable à la vapeur.

6. Emplâtre ou timbre médical suivant les revendications 1 à 5, caractérisé en ce que, dans le cas de l'emplâtre ou timbre médical, il s'agit d'un emplâtre ou timbre collant, d'un bandage, d'un pansement rapide pour plaies ou lésions, d'un emplâtre ou timbre fixable, d'un emplâtre ou timbre d'essai d'allergie, ou d'un pansement étanche aux bactéries.

7. Emplâtre ou timbre médical suivant la revendication 6, caractérisé en ce qu'il est stérilisable.

8. Procédé de fabrication d'un emplâtre ou timbre médical suivant l'une quelconque des revendications 1 à 7, caractérisé en ce que l'on revêt un article plat textile et/ou une feuille perméable à la vapeur, à l'aide d'un outil d'application par héliogravure ou rotogravure pouvant être chauffé qui, lors de la prise de la colle utilise aussi bien une raclette rigide qu'également une raclette élastique, par de la colle de contact fusible, à base de copolymères séquencés de façon poreuse en sorte que la masse de colle forme, en direction longitudinale et en direction transversale, des nervures cohérentes, qui enferment des parties plates ou planes en forme d'îlots et sans colle, qui présentent mutuellement une distance allant jusqu'à 3 mm, où la fraction des parties planes ou plates sans colle forme de 30 à 60%, de préférence, de 40 à 57%, de la surface globale et les nervures de colle sont larges d'au maximum 3 mm à leur base et en ce que l'emplâtre ou timbre présente une perméabilité à la vapeur d'eau d'au moins 2.000 g/m²/24 h, dans les conditions de mesure, où le grammage appliqué de la colle fluctue de 30 à 160 g/m², de préférence, de 40 à 120 g/m².

9. Procédé suivant la revendication 8, caractérisé en ce que l'on utilise, à titre de colle, un copolymère séquencé d'une viscosité à 120-180°C comprise entre 1.000 et 20.000 mPa.s, de préférence, entre 2.000 et 10.000 mPa.s.
